(19) Europäisches Patentamt
European Patent Office
Office européen des brevets

(11) **EP 3 067 693 A1**

(12) # EUROPEAN PATENT APPLICATION

(43) Date of publication:
**14.09.2016 Bulletin 2016/37**

(51) Int Cl.:
*G01N 33/487* [(2006.01)]    *B81B 1/00* [(2006.01)]

(21) Application number: **15158894.4**

(22) Date of filing: **12.03.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(71) Applicant: **Ecole Polytechnique Fédérale de Lausanne (EPFL)**
**1015 Lausanne (CH)**

(72) Inventors:
• **Feng, Jiandong**
  **1110 Morges (CH)**
• **Liu, Ke**
  **1022 Chavannes-Pres-Renens (CH)**
• **Radenovic, Aleksandra**
  **1025 St. Sulpice (CH)**

(74) Representative: **reuteler & cie SA**
  **Chemin de la Vuarpillière 29**
  **1260 Nyon (CH)**

(54) **Nanopore forming method and uses thereof**

(57)    The invention relates to a method for making nanopores in thin layers or monolayers of transition metal dichalcogenides that enables accurate and controllable formation of pore within those thin layer(s) with subnanometer precision. The nanopore is formed by applying a DC transmembrane voltage while monitoring the ionic current through the membrane. When the ionic current reaches a given threshold, the transmembrane voltage is turned off.

Figure 2

## Description

### Field of the Invention

The present invention pertains generally to the fields of nanopore forming in ultrathin membranes based on two-dimensional materials, in particular for use in molecular sensing devices, more particularly solid-state sensing of biomolecules such as DNA, RNA and proteins.

### Background of the Invention

Solid state nanopore bio-sensing is emerging as a rapid single molecule sensing technique (Branton et al., 2008, Nature Biotechnology, 26, 1146; Decker, 2007, Nature Nanotechnology 2, 209). Conceptually, a single nanometer size aperture located on a membrane can detect electrophoretically driven biomolecules translocation in a high throughput manner, revealing localized information of the analyte. However, the formation of single nanopores relies heavily on expensive instrumentation, i.e., Transmission Electron Microscope (TEM) and well trained TEM user, which renders it still confined to laboratory use since this nanopore fabrication process is time-consuming, expensive, not scalable and hard to control at the nm scale.

Further, high costs of the TEM use, coupled with its high initial investment and the time consuming pore drilling process (1 hour machine and operator time per device) limit the more extensive application of solid state nanopores in the bio-sensing field. In addition, not all TEM drilled nanopores are hydrophilic and functional for the sensing of biomolecules. In addition, interaction with the high energy electron beam can cause damage especially when dealing with membranes in 2 D materials.

Many efforts, such as chemical wet-etching of silicon (Park et al., 2007, Small 3, 116-119) or polyethylene terephthalate film (Siwy et al., 2002, Physical review letters 89, 198103) have been carried out towards mass production of nanopores. Recently, a facile method has been reported using dielectric breakdown to make individual nanopores (3-30 nm diameter) on insulating silicon nitride membranes (5-30 nm thick) without the need of TEM (Kwok et al., 2014, Plos One 9, doi:10.1371/journal.pone.0092880, WO 2013/167952) for the *in-situ* forming of nanopores. However, those techniques based on dielectric breakdown need to apply high voltages pulses to the membranes which should be as short as possible for trying to monitor the nanopore diameter during its formation (WO 2014/144818). When reaching dielectric breakdown, the process of pore forming becomes rather uncontrollable which is problematic for reproducibility and quality control of the nanopore size, especially when formed *in-situ* in a nanopore bio-sensing device, thereby leading to important production waste if the quality of the pore does not correspond *in fine* to the prescribed parameters.

Atomically thin nanopore membranes, graphene (Garaj et al., 2010, Nature, 467, 190) and molybdenum disulphide ($MoS_2$) (*Feng et al., 2014, in review*) have drawn much attention recently due to their unprecedented single nucleotide resolution and holds promise as a candidate for so called $3^{rd}$ generation DNA sequencers. Therefore, if fabrication of nanostructures with sub-nanometer, or even single-atom precision has been a long-term goal for nanotechnology in general, there is now a raise of interest for those thin nanopore membranes and an increased need for cost-effective and reliable techniques for nanopore formation in those membranes.

In particular, since the differentiation of biomolecules relies strongly on the pore diameter, there is a high need for developing methods allowing controllable nanopore fabrication, which would enable mass production nanopore in 2D membranes such as $MoS_2$ even below 4 nm with atomic precision.

### Summary of the Invention

An object of this invention is to provide a method for making nanopores in thin nanopore membranes of transition metal dichalcogenide that enables accurate and controllable formation of pore with sub-nanometer precision.

It is advantageous to provide a method of nanopore formation where the pore formation can be carried out *in situ,* notably in a nanopore bio-sensing device.

It is advantageous to provide a method of nanopore formation where the size of the pore is monitored during pore formation and adapted on demand, depending on the sizing need for the different applications such as the type of biomolecules to be sensed (e.g. proteins, or DNA-protein complexes, or nucleotides).

It is advantageous to provide a system for nanopore formation that is economical to implement for mass production and easy to use.

It is advantageous to provide a system for nanopore formation that allows the formation of a broad series of nanopore in parallel on different membranes.

Objects of this invention have been achieved by providing a method according to claim 1.

Disclosed herein, according to a first aspect of the invention, is a method for forming a nanopore in a membrane of transition metal dichalcogenide (TMDC) crystals comprising the steps of:

- providing a TMDC thin layer having from about 0.3 nm to 2 nm thickness ($H_m$) immersed in an electrically conducting liquid ;
- applying a transmembrane voltage (V) at a value higher than the oxidation potential of the transition metal of the said TMDC to the said TMDC thin layer;
- measuring the ionic current ($I_i$) in the said electrically conducting liquid;
- turning off the transmembrane voltage once the measured ionic current ($I_i$) has reached a value ($I_p$) corresponding to the electrical conductance of a pore in the TMDC thin layer having a prescribed diameter ($d_p$).

The applied transmembrane voltage is preferably an essentially constant DC voltage applied between electrodes located on opposing sides of the TMDC thin layer.

In an embodiment, the transition metal dichalcogenide (TMDC) is of chemical formula is $MX_2$, where M is a transition metal atom and X is a chalcogen (S, Se, or Te).

According to a particular embodiment, the TMDC is selected from $MoS_2$, $SnSe_2$, $WS_2$, $TeS_2$, $MoSe_2$, $WSe_2$, and $TeSe_2$.

According to a particular aspect, the TMDC thin layer can be employed in single, double or multilayer form.

According to a further particular aspect, the TMDC thin layer may be a monolayer.

According to a particular embodiment, the TMDC thin layer is suspended in an electrically conducting liquid in such a way that a portion of the thin layer extends over a support layer and the other portion of the thin layer is in contact of a support layer.

In an embodiment of the invention, the material of the support layer may comprise $SiN_x$, glass, $Al_2O_3$ or $HfO_2$.

According to another further particular embodiment, the membrane of transition metal dichalcogenide (TMDC) crystals is a single-layer.

According to an advantageous embodiment, the TMDC layer comprises $MoS_2$ thin layers or is a $MoS_2$ monolayer.

According to an advantageous embodiment, the TMDC layer comprises CVD grown thin layers or is a CVD grown monolayer.

In an embodiment of the invention, the electrically conducting liquid may advantageously comprise or consist in an aqueous liquid comprising an electrolyte.

In an embodiment of the invention, the electrically conducting liquid may be different from each side of the transition metal dichalcogenide membrane, in particular when the nanopore formation process is to be conducted *in situ* in a nanopore sensing device.

In an embodiment of the invention, the electrolyte may be potassium chloride (KCl).

In an embodiment of the invention, the electrically conducting liquid is an aqueous ionic solution (e.g. water and KCl or any inorganic salts such as LiCl, NaCl, $MgCl_2$ $CaCl_2$ etc).

In another embodiment, the transmembrane voltage is applied in a continuous manner.

In another embodiment, the transmembrane voltage is applied for a period of time necessary to reach an ionic current value which corresponds to the electrical conductance of a pore having a prescribed diameter (prescribed current/conductance).

In an embodiment of the invention, the ionic current can be measured in an ionic current circuit comprising a pair of electrodes (e.g. Ag/AgCl) coupled to the conducting liquid on opposite sides of the membrane.

In an embodiment, the ionic current circuit comprises means to measure the ionic current configured to provide a signal used in the measurement of the conductance through the nanopore.

According to another embodiment, the turning off of the transmembrane voltage is achieved by an automatic switch which is activated through a feed-back control circuit when a prescribed current/conductance is reached.

According to another embodiment, the oxidation potential of a transition metal dichalcogenide can be determined by cyclic voltametry. For example, a voltage of 800 mV is higher (typically between 800 mV and 1'000 mV such as about 800 mV and 900 mV) than the oxidation potential of $MoS_2$ being oxidized to $Mo^{(VI)}$ and allows starting the ECR process.

According to a particular embodiment, once the ECR process starts, the transmembrane voltage is applied at a potential slightly lower than oxidation potential for the bulk material, for example about 5% lower the oxidation potential (e.g. about 5% ($\pm$ 1 or 2%) lower). This allows the manufacture of a single pore at a vacancy instead of making several pores outside such vacancy.

According to a particular embodiment, the transmembrane voltage is applied at a potential slightly higher than oxidation potential (e.g. about 5 to 10% higher) and once the ECR process starts, the transmembrane voltage is applied at a potential slightly lower than oxidation potential for the bulk material, for example about 5 $\pm$ 1 or 2% lower the oxidation potential.

According to another embodiment, is provided a method of manufacturing a nanopore sensing device comprising a step of forming a nanopre according to the invention.

According to a further embodiment, is provided a method of manufacturing a nanopore sensing device according to the invention further comprising a step, once the nanopore is formed, of exchanging the electrically conducting liquid in the *cis* side of the transition metal dichalcogenide membrane by an electrically conducting liquid comprising a room temperature ionic liquid (RTIL).

In a further embodiment of the invention, the room temperature ionic liquid (RTIL) is selected from an essentially pure RTIL, optionally mixed with an organic solvent, or a mixture of a water-miscible RTIL in water with a water content from about 5 to about 50 wt%.

According to an embodiment, is provided a method according to the invention, wherein more than one TMDC thin layers are provided and a transmembrane voltage is applied to each of the TMDC thin layers in parallel.

The above mentioned features may be combined in any appropriate manner.

An advantageous characteristic of the invention is to provide a method where the pore characteristics such as size, shape, and edge properties are fully controllable and reproducible.

An advantageous characteristic of the invention is to provide a method where the pore formation process lasts for few minutes of less.

An advantageous characteristic of the invention is to provide a method where the formation of the nanopore(s) in the transition metal dichalcogenide membrane is achieved by atomically controlled electrochemical etching of transition metal dichalcogenide thin layer, including monolayers with a sub-nanometer precision

According to one aspect, the method of the invention allows *in-situ* preparation of nanopores in membranes of a transition metal dichalcogenide by electrochemical reaction (ECR), in particular in a nanopore sensing device.

An advantageous characteristic of the invention is to provide a method where pore dimensions may be adjusted to the needs, in particular to the biomolecule to sense in a nanopore sensing device.

A noticeable advantage for a nanopore fabrication method of the invention is that biomolecule translocations can be performed *in situ* directly after ECR and size-control allows on-demand adaptation of the pore size, allowing sizing for the different types of biomolecules, e.g. proteins or DNA-protein complexes etc...

According to a further aspect, the method can be applied to a plurality of transition metal dichalcogenide membranes using a multiple channels feedback control unit for monitoring the ionic current through a plurality of transition metal dichalcogenide membranes.

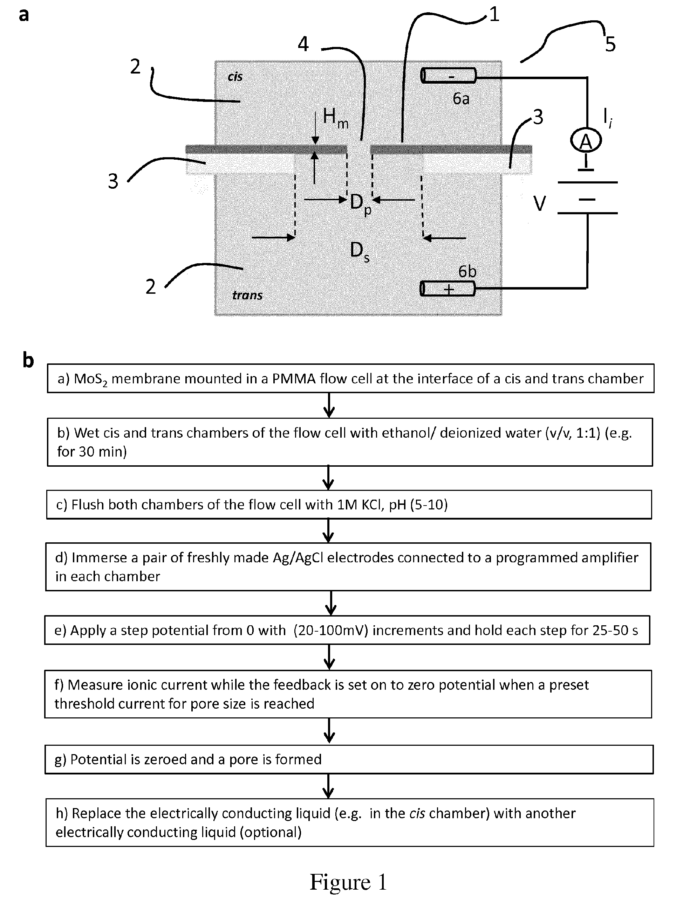

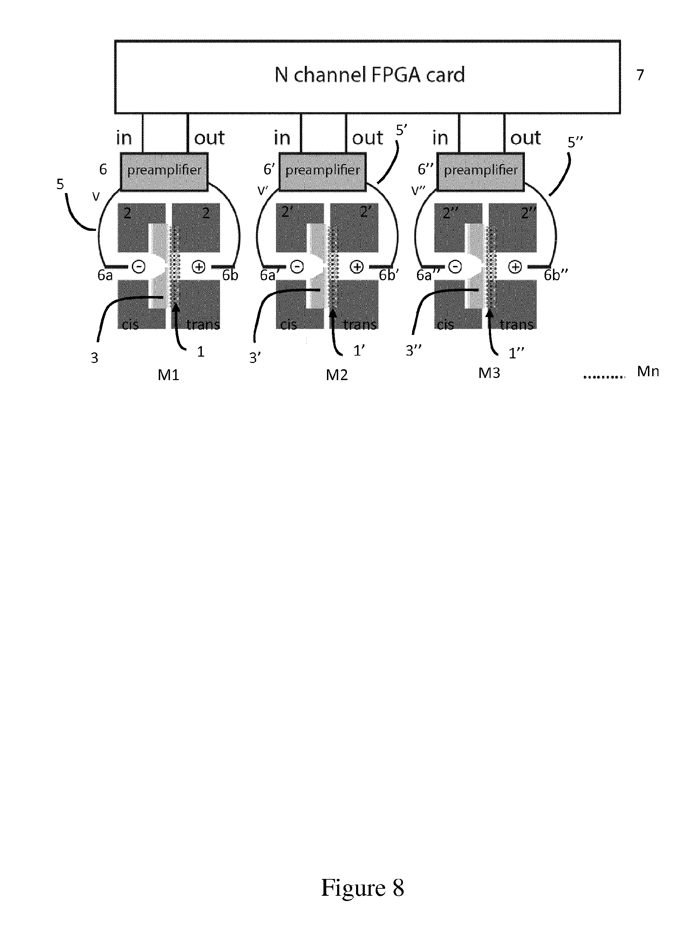

Referring to Figure 8, is illustrated a method for forming a nanopore in a transition metal dichalcogenide membrane according to the invention wherein the transition metal dichalcogenide membrane is in a form of a thin layer (or a monolayer) 1 having from about 0.3 nm to 4 nm thickness as depicted in Figure 1 wherein more than one TMDC thin layers (1, 1' and 1") are provided and a transmembrane voltage is applied to each of the TMDC thin layers in parallel (V, V' and V"). Each TMDC thin layer is suspended in an electrically conducting liquid (2, 2', 2") and each transmembrane voltage is applied at a value higher than the oxidation potential of the transition metal of the corresponding TMDC, measuring the ionic current ($I_i$) in the said electrically conducting liquid until the ionic current ($I_i$) has reached a value ($I_p$) corresponding to the electrical conductance of a pore within the metal dichalcogenide membrane having a prescribed diameter ($D_p$). The TMDC thin layer 1 is configured such that the portion of the TMDC thin layer where the formation of a nanopore will be carried out is suspended in an electrically conducting liquid and the other portion of the TMDC thin layer is supported by a support layer (3, 3' and 3 ").

A multiple channels feedback control unit 7 (e.g. N channel FGPA card) is used for achieving in parallel (a) monitoring transmembrane voltage through each of the TMDC thin layers, (b) monitoring the recording of the measurement of the ionic current in an electrical circuit (5, 5', 5") formed between a voltage source V (V', V") and electrodes 6a and 6b (6a", 6b"; 6a', 6b") immerged into the electrically conducting liquid on both sides of the corresponding TMDC thin layer 1, 1', 1" and (c) the turning off the transmembrane voltage when a prescribed current/conductance is reached for the corresponding TMDC thin layer. The different thin layers (1, 1' and 1") can be provided in the form of thin membrane devices M1 to Mn mounted in parallel. A preamplifying unit for each of electrical circuit (6, 6', 6") can be connected to the multiple channels feedback control unit 7 and the voltage sources.

According to an embodiment, by for instance using a field programmable gate array (FPGA) board (NI PXI-7851R), the manufacture of a plurality of nanopores, for instance eight, can be monitored in parallel.

Moreover, a method according to the invention could also pave the way to cheaper sensing devices by taking advantage of wasteless *in situ* nanopore forming adapted for scale up production of 2D nanopores and shrink the costs for sensing devices based on such nanopore membranes.

Other features and advantages of the invention will be apparent from the claims, detailed description, and figures.

**Brief Description of the drawings**

Figure 1 is an illustration of exemplary settings for carrying out the process of the invention. **a**: disposition of the 2D transition metal dichalcogenide for the *in situ* formation of a nanopore. **b**: flowchart illustration of the steps of a process of the invention as implemented according to an exemplary embodiment of Example 1.

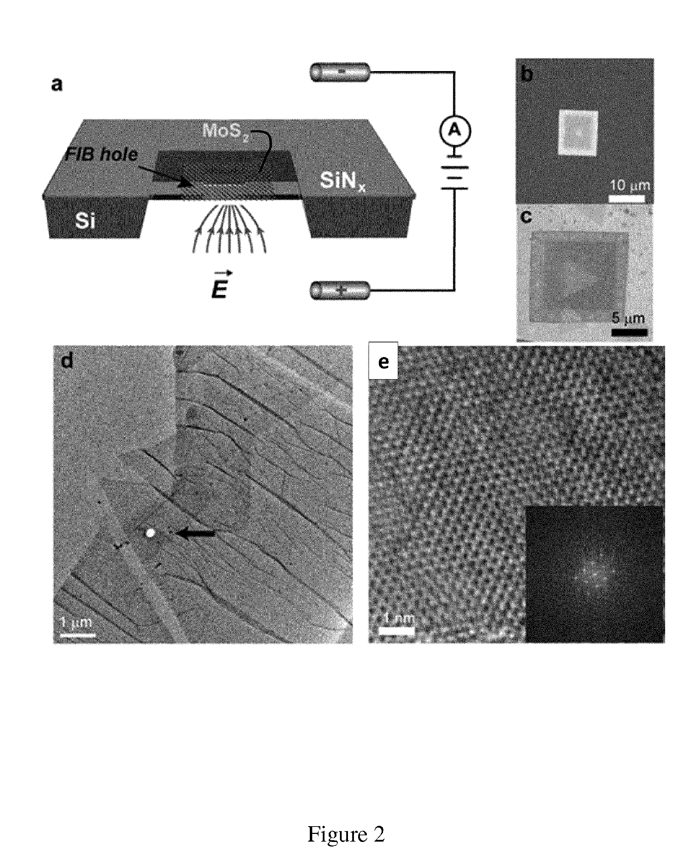

**Figure 2** is an illustration of the use of a method of the invention for in situ fabricating nanopores in a transition metal dichalcogenide membrane in a molecular sensing system as described in Example 1. **a:** Schematic illustration of preparation of a freestanding $MoS_2$ membrane ready for electrochemical formation of a nanopore. In the center of the supporting 20 nm thick $SiN_x$ membrane a single focused ion beam, focused ion beam (FIB) hole is drilled to suspend a small portion of an intact monolayer $MoS_2$ flake. A single chip is mounted in the flow-cell for typical translocation experiments. A pair of electrodes (e.g. Ag/AgCl) is connected to a preamplifier is used to apply transmembrane voltage; **b:** Optical image of the SiNx membrane with a FIB drilled hole in the center. c: Optical image of the $SiN_x$ membrane with transferred triangular CVD-grown $MoS_2$ monolayer; d: Low magnification TEM image of transferred CVD-grown $MoS_2$ monolayer covering the FIB hole. The FIB hole is indicated by the black arrow; e: High resolution TEM of the lattice of $MoS_2$ suspended over the FIB hole. The corresponding diffraction diagram is shown in the inset

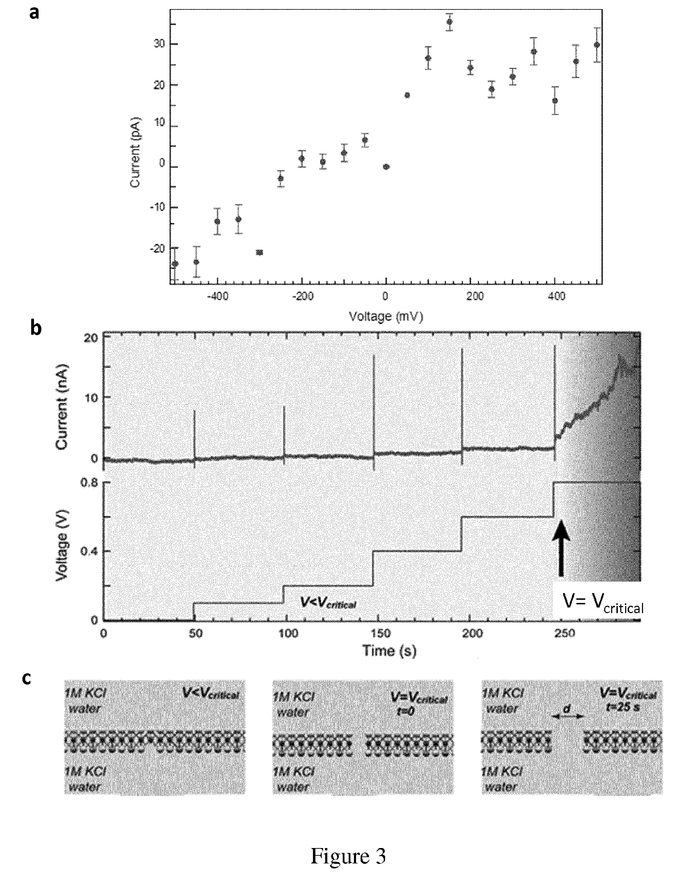

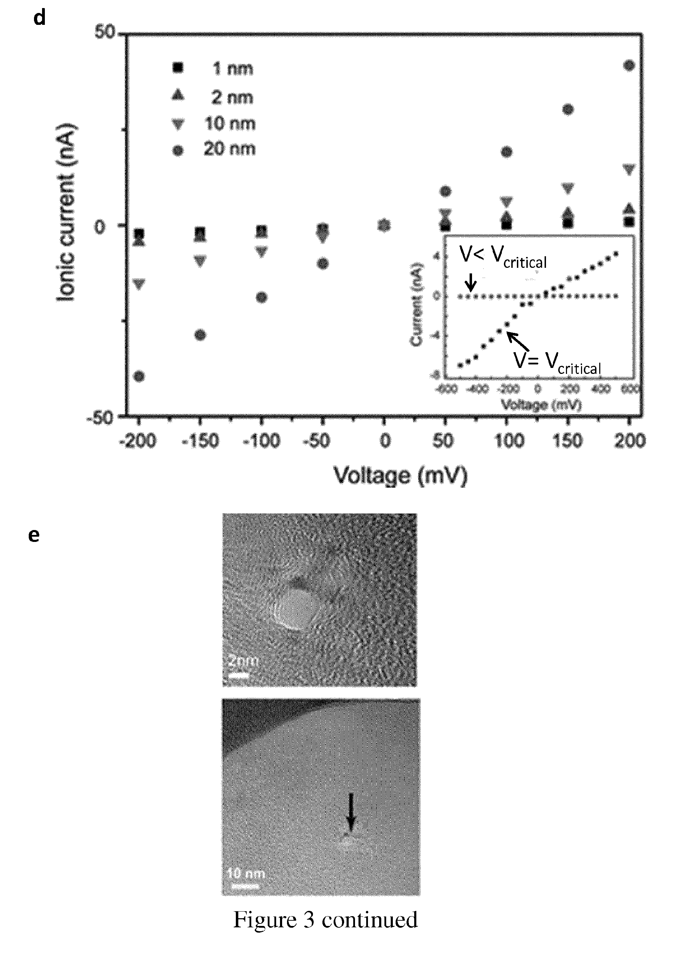

**Figure 3** shows the leakage current characteristics of an intact membrane and during pore formation as described in Example 1 together with the visualization and modelization of the pore forming process. **a**: Leakage current-voltage (I-V) characteristic of an intact $MoS_2$ membrane, for voltages below the critical voltage of 800 mV required for ECR which depends on the number of the membrane defects (more defects leads to higher current). **c**: Representative ionic current trace measured for a $MoS_2$ membrane. Voltage is stepped by 100 mV with a 50 s holding, and the leakage current increases in accordance, being steady for a constant voltage. Sharp peaks at each voltage step originate from the capacitance charging. After a critical voltage, 800 mV is applied, the electrochemical reaction (ECR) starts (arrow), the current keeps increasing which triggers the feedback control to switch off voltage bias in order to halt the pore growth. **c**: Mechanism of ECR based $MoS_2$ nanopore fabrication. A side view of the monolayer $MoS_2$ lattice, emphasizing the lattice having single atom (S) vacancy or defect before ECR V<Vcritical (left), $MoS_2$ lattice at V=Vcritical (nanopore starts to form, middle) and $MoS_2$ lattice when nanopore (diameter d) is formed (right). **d**: Current-voltage (IV) characteristic of nanopores ranging in diameter from 1 to 20 nm - all nanopores are created via electrochemical reaction. Inset shows I-V characteristics for the system below and at the critical voltage. **e**: TEM images verify the nanopore formation and size (top being a zoom-in image of the bottom image).

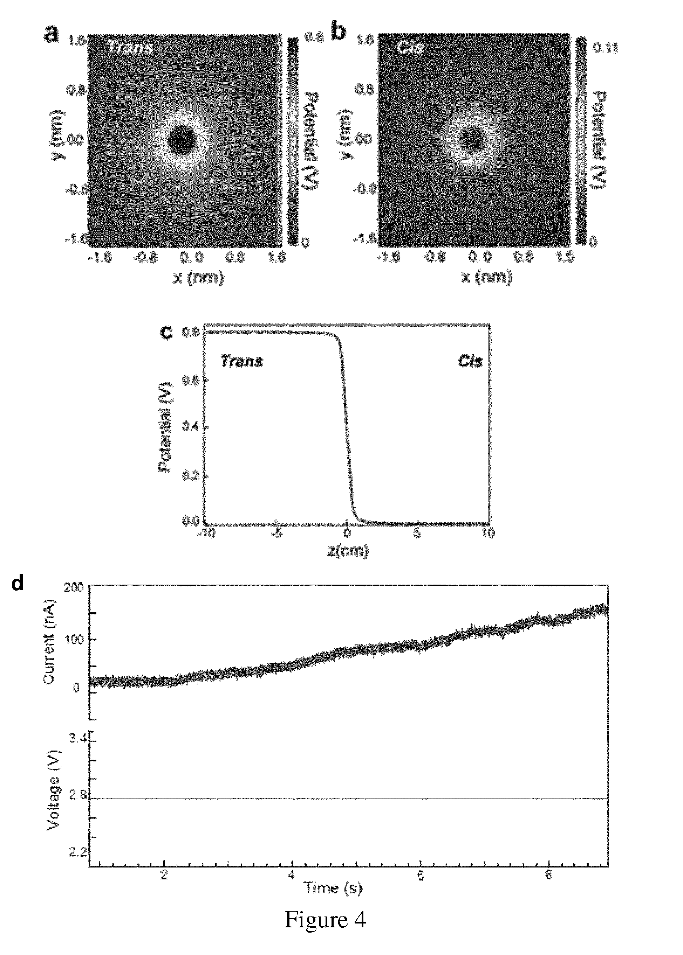

**Figure 4** shows simulations of the electric potential distribution for the nanopore in two dimensions for a freshly formed pore having a diameter of 0.3 nm and comparison with a typical current trace of nanopore formation on graphene membrane. **a**: Electric potential distribution in the *trans* chamber in the immediate vicinity of the membrane surface and **b**: in the *cis* chamber. **c**: Electric potential distribution as a function of the distance from the pore. The applied potential was set to 800 mV and salt concentration was 1 M KCl. **d**: Typical current trace of nanopore formation on graphene membrane using ECR.A much higher transmembrane voltage, 2.8 V has to be applied to graphene to create a nanopore in graphene.

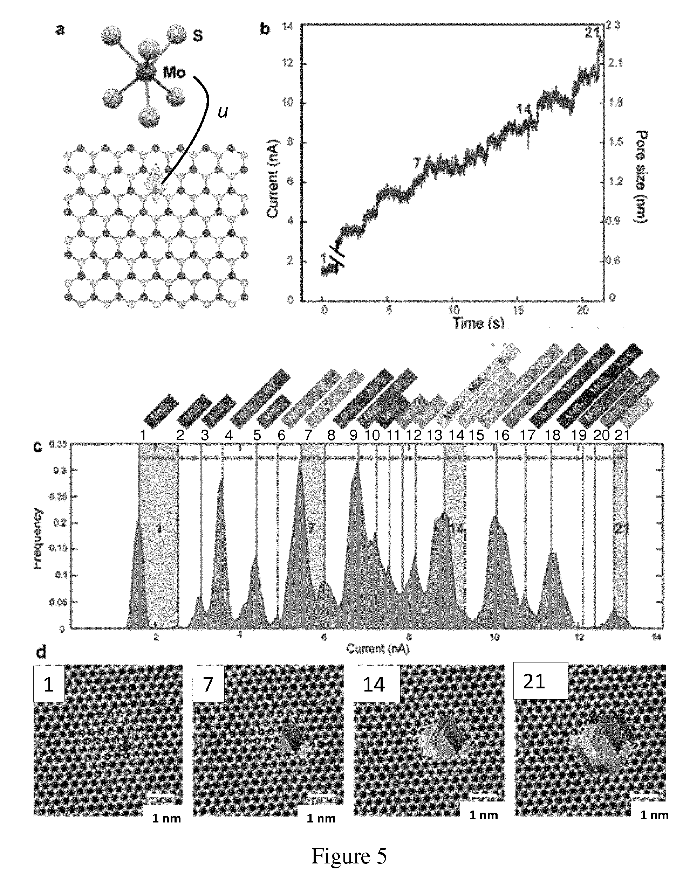

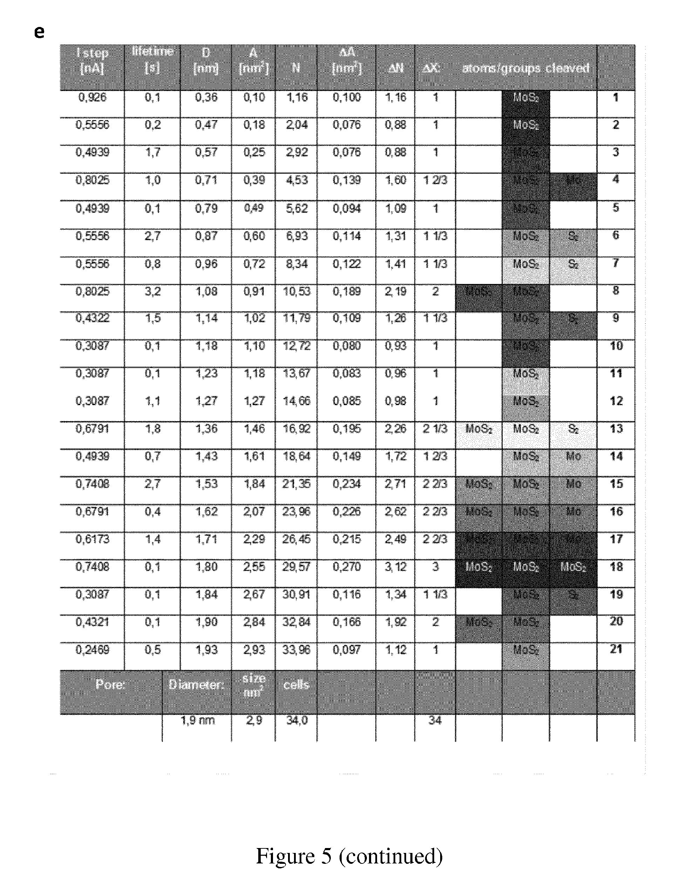

**Figure 5** is schematization of nanopore forming in monolayer $MoS_2$ starting from a monolayer $MoS_2$ lattice **a:** Top view of the monolayer $MoS_2$ lattice, the unit cell, u (parameter a=3.12 Å) is shown in grey *(Hulliger, & Lévy. Structural Chemistry of Layer-Type Phases, Springer, 1976)*. **b**: Ionic current-steplike features during the nanopore formation in **Fig. 2a. c:** Custom Matlab code is used to detect steps in the raw ionic current trace (Raillon et al., 2012, Nanoscale, 4, 4916-4924 and histogram of the trace shown in b) with corresponding color coded atom groups cleaved in each step during the pore formation from 1 to 21. **d:** Illustrative schematic of polygon removal corresponding to the histogram trace. Aberration corrected TEM micrograph of suspended single layer $MoS_2$ superimposed are the polygons which correspond to atom groups cleaved in the steps 1, 7, 14 and 21 during the pore formation. The coloring of atom groups cleaved in each step **(Fig. 5c)** and corresponding area polygons shown in the Fig. 5d starts from step 1 and goes up to step 21. e: The table represents the sequence of cleaving $MoS_2$ unit cells and Mo and S atoms in 21 steps to form the pore. $I_{step}$ is the distance between two adjacent peaks in the current histogram; D and A are effective pore diameter and pore area, respectively; N is the number of unit cells equivalent to the effective area; $\Delta A$ is the increments of A; $\Delta N$ is the increments of N; $\Delta X$ is the nearest integer or integer +1/3 or +2/3 of $\Delta N$; the last column on the right stands for the number of unit cells.

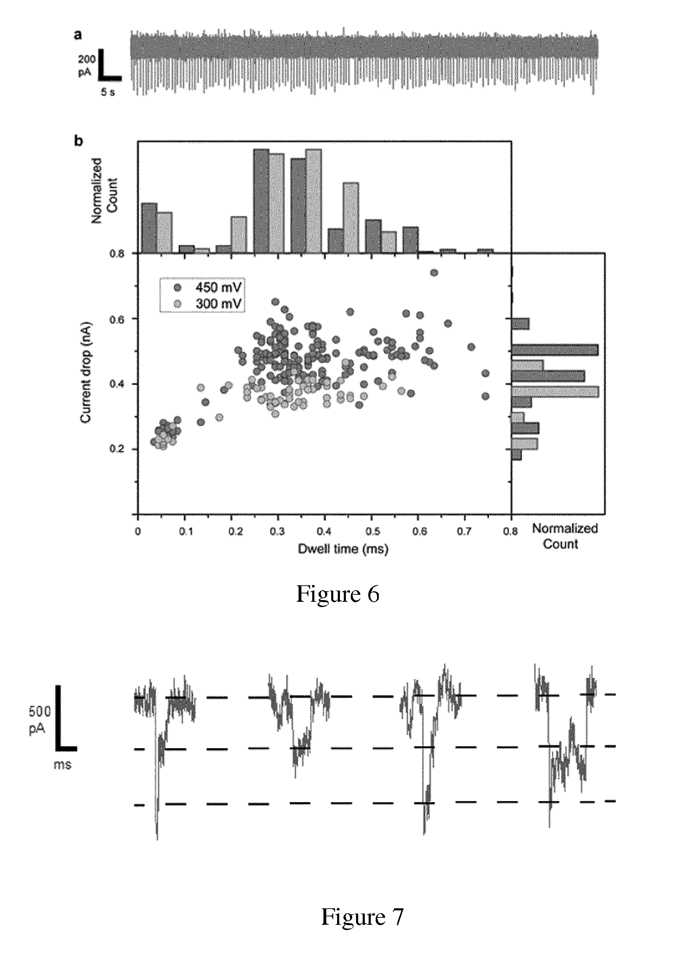

**Figure 6** shows DNA translocation through a nanopore fabricated as described in Example 1. **a:** typical trace of pNEB plasmid DNA translocation through an electrochemically etchhed nanopore recorded at 450 mV. The trace is downsampled to 10 kHz for display. **b**: Scatter plot of events collected at 300 mV and 450 mV bias. Event detection is performed using OpenNanopore37 Matlab code. Expectedly, the increase in the bias voltage from 300 to 450 mV shortens the translocation time and enhances the current drop.

**Figure 7** shows selected events of λ-DNA translocation through a 4 nm ECR fabricated $MoS_2$ nanopore by a method according to the invention as described in Example 1 and recorded *in-situ* right after pore formation at 200 mV as described in Example 2. Quantized levels in the ionic current represent the flexibility of λ-DNA when translocating through nanopore.

**Figure 8** shows and example of arrangement of setting for carrying out the process of the invention for the *in situ* formation of a nanopore on several devices comprising a 2D transition metal dichalcogenide membrane in parallel through the control of an integrated circuit configured for the monitoring of the application and cut-off (e.g. N- channel FPGA card) transmembrane voltages in parallel to each of the 2D transition metal dichalcogenide membranes.

**Detailed Description of the invention**

Referring to the figures, in particular first to Figures 1a and 2, a method for forming a nanopore in a transition metal dichalcogenide membrane, in a form of a thin layer (or a monolayer) 1 having from about 0.3 nm to 4 nm thickness ($H_m$) suspended in an electrically conducting liquid 2 comprises applying a transmembrane voltage (V) at a value higher than the oxidation potential of the transition metal of the said TMDC, measuring the ionic current ($I_i$) in the said electrically conducting liquid until the ionic current ($I_i$) has reached a value ($I_p$) corresponding to the electrical conductance of a pore within the metal dichalcogenide membrane having a prescribed diameter ($D_p$).

The TMDC thin layer 1 is configured such that the portion of the TMDC thin layer where the formation of a nanopore will be carried out is suspended in an electrically conducting liquid and the other portion of the TMDC thin layer is supported by a support layer 3.

The support layer can be of $SiN_x$, glass, quartz, $Al_2O_3$, or $HfO_2$ (or any other material that provides low capacitance membrane) with a support orifice of a diameter $D_s$ that allows the portion of the TDMC membrane where the pore formation should be conducted being suspended in the electrically conducting liquid.

When the transmembrane voltage V is applied to the TMDC thin layer at a value higher than the oxidation potential of the transition metal of the said TMDC, TMDC start being oxidized through an ECR reaction and the formation of a nanopore 4 is initiated.

The formation for a nanopore 4 could be monitored through the measurement of an increase in the ionic current ($I_i$) in an electrical circuit 5 formed between a voltage source V and electrodes 6a and 6b immerged into the electrically conducting liquid on both sides of the TMDC thin layer 1, and configured to measure the ionic current $I_i$ by a current measurement circuit portion A. The current measurement circuit portion A measures the ionic current value which is representative of conductance through the pore in the TMDC thin layer 1 and therefore of the diameter Dp of the pore 4 formed in the TMDC thin layer 1.

Thin layers of $MoS_2$ with good quality suitable for use in a device according to the invention can be prepared by both exfoliation and chemical vapor deposition (CVD) (Novoselov et al., PNAS, 2005, 102, 10541-1053; Liu et al. 2012, Nano Lett., 12, 1538-1544).

Typically, the thickness of TDMC membranes according to the invention can be assessed by Raman /optical electron microscopy, photo-luminescence (PL) measurements and Atomic Force Microscopy (AFM).

According to another particular aspect, the thickness of a TDMC membrane according to the invention may be less than 2 nm, typically from about 0.7 nm to less than 2 nm. In particular, the TDMC membrane is from about 0.7 nm (e.g. one layer) to about 1.4 nm thick (e.g. two layers).

According to another aspect, pores in the TDMC membrane formed by a process of the invention are nanometer sized, typically from about 1 nm to 20 nm diameter (for example typically from about 1 nm to about 5 nm, for example less than 4 nm or less such as about 3 nm) and from about 0.3 nm to

1 nm thickness (for example about 0.7 nm). Typically, the size of the pores can be measured by Transmission electron microscopy (TEM) and calculated from the current -voltage characteristics.

According to another aspect, the support layer can be a $SiN_x$, glass, or quartz (or any other material that provides low capacitance membrane) with a support orifice that allows the portion of the TDMC membrane where the pore formation should be conducted being suspended in the electrically conducting liquid. According to a further aspect, the support orifice has typically a diameter of from about 20 nm to about 500 nm (e.g. 50 nm), like for example from about 200 nm to about 500 nm and from about 20 nm to 50 nm thick.

According to a further aspect, the support layer can be coated with some curing layer such as polydimethylsiloxane (PDMS), while leaving the $MoS_2$ nanopore exposed in order to reduce the dielectric noise. Alternatively, support layer can be a quartz, glass, or any other material that provides low capacitance membrane based support.

According to a particular embodiment, the TDMC thin layer of the invention can be an active layer as described in WO 2012/093360.

According to a particular embodiment, the method of the invention can be carried out in a nanopore sensing device as generally described in PCT/EP2015/053042.

Referring to Figure 1b, is provided an illustration of a specific embodiment regarding steps of a method for forming a nanopore in a transition metal dichalcogenide membrane according to the invention:

(a) providing a TMDC thin layer mounted in a housing (e.g. polymethylmethacrylate (PMMA)) flow cell chamber on a support layer (e.g. $SiN_x$) with a support orifice that allows the portion of the TDMC membrane where the pore formation will be conducted being suspended in the electrically conducting liquid, wherein the housing comprises a *cis* and a *trans* chamber and the TMDC thin layer is located at the interface of those two chambers;

(b) filling *cis* and *trans* chambers with an aqueous solution (e.g. a mixture of ethanol/deionized water v/v, 1:1) for increasing its hydrophilicity (e.g. for about 15-35 min);

(c) flushing the *cis* and *trans* chambers with an electrically conducting liquid (e.g. 1M KCl) such as the TMDC thin layer is immersed in said electrically conducting liquid;

(d) immersing a pair of electrodes adapted to the electrically conducting liquid (e.g. freshly made Ag/AgCl electrodes) in the corresponding chambers and connected to a voltage source, for example through a programmed amplifier;

(e) applying a transmembrane voltage by step-wise increments (e.g. 20-100 mV increments) and holding each step for about 25-50s;

(f) measuring the ionic current ($I_i$) in the said electrically conducting liquid;

(g) turning off the transmembrane voltage (i.e. by feed-back control) once the measured ionic current ($I_i$) has reached a value ($I_p$) corresponding to the electrical conductance of a pore in the TMDC thin layer having a prescribed diameter ($d_p$).

(h) optionally replacing the electrically conducting liquid in the *cis* chamber with another electrically conducting liquid (e.g. suitable for nanopore biosensing).

Apart from nanopore sensors, other applications for a method according to the invention can be envisioned such as water desalination devices such as described in Yuan & Gaoquan, 2014, Nanoporous graphene materials, Materials Today, 17 (2) 77-8 and Cohen-Tanugi Det al., 2012, 2012, Nano Letters, 12, p. 3602-3608.

The invention having been described, the following examples are presented by way of illustration, and not limitation.

## Examples

### Example 1: Preparation of MoS$_2$ nanopores using ECR

The present example illustrates a method of the invention applied to the fabrication of individual nanopores on single-layer MoS$_2$, with the electric field generated by Ag/AgCl electrodes located in two electrolytes compartments and positioned away from the membrane. As described below, the ECR process starts for a certain critical voltage applied to the membrane at a defect/vacancy present in the MoS$_2$ membrane.

Importantly, in the course of the ECR process, it is possible to control the successive removal of single or few MoS$_2$ units from the monolayer MoS$_2$ membranes. In this way, atom-by-atom nanopore engineering is achieved.

A procedure for fabricating MoS$_2$ nanopores using ECR is schematically illustrated in **Fig. 2a,** where two chambers (*cis* and *trans*) are filled with aqueous buffer (1M KCl, pH 7.4) and biased by a pair of Ag/AgCl electrodes which are separated by a single-layer MoS$_2$ membrane. Presence of an active site such as single-atom vacancy facilitates the removal of individual atoms and MoS$_2$ unit cells from MoS$_2$ lattice by ECR at voltages higher than the oxidation potential of MoS$_2$ in aqueous media. This process is facilitated by the electric field focusing by the pore itself. To form freestanding membranes, CVD-grown monolayer MoS$_2$ transferred from a sapphire substrate is suspended over focused ion beam (FIB) defined openings that ranged from 80 nm to 300 nm in diameter and were centered in a 20 nm thick SiN$_x$ membrane **(Fig. 2b).** A typical optical image of the transferred triangular flake of CVD-grown monolayer MoS$_2$ on the supporting silicon nitride membrane is shown in **Fig. 2c.** The freestanding MoS$_2$ membrane above the FIB defined opening can be further identified under TEM with low magnification (5 k$\times$) as shown in **Fig. 2d.** MoS$_2$ flake is further characterized by Energy-dispersive X-ray spectroscopy (EDX) in TEM to reveal the chemical composition of the surface where elements of Mo and S are abundant in triangular areas. When moving to a high magnification (1 M $\times$) and focusing on the freestanding portion of MoS$_2$ over the FIB opening, the atomic structure of MoS$_2$ can be clearly resolved as shown in **Fig. 2e,** and the diffractogram reflects the hexagonal symmetry of MoS$_2$, as shown in the inset of **Fig. 2e.**

An intact MoS$_2$ membrane is mounted into a custom made microfluidic flow-cell filled with an aqueous buffer and transmembrane potential is applied using a pair of Ag/AgCl electrodes as shown in **Fig. 2a.** When a voltage is applied below the potential for electrochemical oxidation of the transition metal of the membrane, small leakage current is normally detected, typically on the order from tens to hundreds of picoamperes depending on the number of defects in the 2D membrane. As shown in **Fig. 3a,** the leakage current displays a non-ohmic characteristic. To reach the critical voltage value for achieving an ECR, the potential is gradually stepped, as shown in **Fig. 3b.** When the applied voltage is stepped up to 0.8 V (a critical voltage, indicated by the arrow), an increase of baseline current immediately occurs. This time-point indicates the nanopore creation which is associated to the electrochemical dissolution of MoS$_2$ enhanced by the electrical potential focused on the active site as shown in the potential profile obtained by the finite element analysis simulation **(Fig. 4a).** In other words, the pore growth continues at the initial, active site instead of another start on another defect because the pore itself focuses the applied electric potential and facilitates continuation of ECR on the active site where ECR process has already started.

In contrast to the avalanche-like dielectric breakdown process in silicon nitride, where a typically 10-minute waiting time for the filling of charge traps (Briggs et al., 2015, nanotechnology, 084004, doi:10.1088/0957-4484/26/8/084004) under the application of critical voltage (> 10 V) is needed before breakdown occurs, electrochemical dissolution happens spontaneously at the critical voltage. In addition, the observed rise of ionic current shows a quite slow rate ($\sim$ 0.4 nA/s). Therefore, it is possible to control the nanopore size by using an automatic feedback to cut off the voltage once the desired current/conductance threshold is reached. This feedback also helps to avoid multiple pore formation. Owing to the limited rates of electrochemical reaction, the $MoS_2$ nanopore sculpting process is quite slow, occurring on time scales of dozens of seconds to several minutes. **Fig. 3e** gives an example of ionic current trace to reach the threshold of 20 nA, for the critical voltage of 0.8 V.

Taking the advantage of existing theoretical insights to model the conductance-pore size relation (Kowalczyk et al., 2011, Nanotechnology, 22, doi:Artn 315101 Doi 10.1088/0957-4484/22/31/315101), the conductance of the nanopore (G) can be described by

$$G = \sigma \left[ \frac{4L}{\pi d^2} + \frac{1}{d} \right]^{-1} \qquad (1)$$

where $\sigma$, $L$ and $d$ are the ionic conductivity of solution, membrane thickness and nanopore diameter, respectively. Using this relation in combination with feedback on ECR that immediately stops the voltage once the desired pore conductance - that corresponds to a certain pore size - is reached, it is possible to fabricate pores ranging in diameter from 1-20 nm. **Fig. 3e** reveals current-voltage (I-V) characteristics of $MoS_2$ nanopores fabricated by ECR with different estimated sizes ranging from 1 nm to 20 nm. The symmetric and linear I-V curves also imply the well-defined shape of the fabricated pores. Similarly, as shown in the inset of the **Fig. 3e**, I-V characteristics across the membrane have been investigated in situ before and after ECR, confirming the pore formation.

To further verify the size of fabricated $MoS_2$ nanopores, TEM has been used to image the newly formed nanopore. Exposure of 2D materials to electron radiation can induce large area damage and also open pores, as reported for both graphene (Fischbein et al., 2008, Applied Physics Letters, 93) and $MoS_2$ (Liu et al., 2013, Nat. Commun., 4). Therefore, first aligned the beam was aligned on the suspended portion of $MoS_2$ outside of the FIB opening and then quickly scanned the sample while taking care to minimally irradiate suspended $MoS_2$ that houses ECR fabricated pore. **Fig, 2c** shows a TEM image of an ECR-fabricated $MoS_2$ nanopore.

For the sake of comparison, a few graphene membranes (prepared as described below) have also been tested by this method and higher voltages (2-3V) are required to fabricate pores as presented in Supporting Information, with the typical ionic current trace is displayed in **Fig. 4b.**

The described ECR-based pore formation method benefits from the unique crystal structure of transition metal dichalcogenide ($MX_2$) where atoms are situated in tree planes and linked by metal-chalcogenide bonds while in the case of graphene, carbon atoms are in the same plane and 3 bonds need to be removed to release one carbon atom. In addition, to remove carbon atoms, graphene needs to be oxidized to a higher valence state which presumably requires a higher voltage.

The physics of the electrochemically fabricated nanopores is determined by the focused electrical field and surface chemistries. The electric field concentrates at surface irregularities or defects which can be considered as surface active sites, and focuses current flow at the site of the pore, and thus locally enhances the electrochemical dissolution, as shown in **Fig 3c.**

The surface dissolution chemistries can be understood as a surface bound oxidation scheme with hole capture and electron injection to produce the $MoS_2$ oxidation state (Bonde et al., 2008, Faraday Discussion 140, 219-231) as shown in

$$MoS_2 + 11H_2O = MoO_3 + 2SO_4^{2-} + 22H^+ + 18e^- \qquad (2)$$

where $MoS_2$ is oxidized into $MoO_3$ which is water-soluble. Without being bound to any theory, this reaction is highly likely to happen considering the electrical potential (voltage bias) range applied to the membrane. Due to the current technical limitations of electron energy loss spectroscopy (EELS) analysis in the nanopore vicinity, it cannot be excluded the possibility that $MoS_2$ is oxidized to other valence states. Once an active site is removed by the process described above, indicating the initial formation of the pore, **Fig. 5a**, the electric field, as simulated in **Fig. 4a**, entirely focuses in this pore, which then prefers to enlarge the same pore since this is more energetically favorable than creating a second pore at another location. By applying at the beginning of the fabrication process a bias voltage higher than the critical voltage it might be possible to observe the formation of multiple pores. Given the stochastic nature of the pore creation process, with a configuration of voltage steps, multiple simultaneous nanoscale ECR events are highly unlikely. Furthermore, feedback control on the applied voltage to obtain the desirable conductance ensures the formation of a single nanopore. Finally, the formation of single nanopore is confirmed by TEM imaging.

The advantage of an ECR-based nanopore fabrication technique of the invention, apart from the benefit of being a fast and cheap production lies in the possibility of fine-tuning the diameter of nanopores with unprecedented, single-atom precision. The low nanopore enlarging speed is due to low voltages and the electrochemical dissolution nature of the process. **Fig. 5d** is a 25-second long, continuous pore conductance trace that shows atomic precision during nanopore sculpting process. The trace starts from the critical point indicated in **Fig. 3b.** Fitting to the conductance-nanopore size relation, it can be estimated a pore diameter growth rate of about 1 angstrom per second. After 25 seconds a pore with a diameter 1.9* nm (area of 2,9 nm$^2$) has been formed. The area of such a pore is equivalent to almost exactly N = 34 unit cells of MoS$_2$ where the area of the unit cell $u$ = 0.0864 nm$^2$ **(Fig. 5a).** *The final pore size is 2,2, nm when one accounts for the initial 0.3 nm.

Surprisingly, the growth curve is not linear but step-like, as shown in **Fig. 5b.** Necessarily, the effective size of the pore enlarges with the same step-like characteristic. These step-like features are commonly observed when working with voltages around 1V. To gain insights into these step-like features, the histogram of current values is plotted from this trace in **Fig. 5c,** where 21 individual peaks can be extracted from the histogram.

The sequence of the pore size enlargement steps may be normalized by the unit cell area $u$ and a sequence of MoS$_2$ formula units and Mo and S atoms cleaved (corresponding to 21 current steps) to form the pore may be inferred, as presented in the **Fig. 5c** and **5e.** Several snapshots of the pore formation process taken at steps 1,7,14 and 21 are displayed in the **Fig. 5d.** The area of polygons corresponding to the cleaved atom groups follows the honeycomb structure of single-layer MoS$_2$, as presented in an aberration corrected TEM image **Fig 5d** and in the schematics shown in **Fig 5a.** The observed atomic steps here reveal the ultimate precision (single atoms) that can be reached in engineering nanostructures with a process of nanopore forming of the invention.

*Membrane set-up*

The MoS$_2$ membranes are prepared using the previously reported procedure (Liu et al., 2014, ACS Nano 8, 2504-2511) and as described below. Briefly, 20 nm thick supporting SiN$_x$ support membranes are manufactured using anisotropic KOH etching to obtain 10 $\mu$m $\times$ 10 $\mu$m to 50 $\mu$m $\times$ 50 $\mu$m membranes, with size depending on the size of the backside opening. Focused ion beam (FIB) is used to drill a 50-300 nm opening on that membrane. CVD-grown MoS$_2$ flakes were transferred from sapphire substrates using MoS$_2$ transfer stage in a manner similar to the widely used graphene transfer method and suspended on FIB opening (Dumcenco et al., 2014, arXiv preprint arXiv:1405.0129). Membranes are first imaged in the TEM with low magnification in order to check suspended MoS$_2$ flakes on FIB opening.

*CVD MoS$_2$ growth*

Monolayer MoS$_2$ has been grown by chemical vapor deposition (CVD) on c-plane sapphire. After consecutive cleaning by acetone/isopropanol/DI-water the substrates were annealed for 1h at 1000 °C in air. After that, they were placed face-down above a crucible containing ~5 mg MoO$_3$ ($\geq$ 99.998% Alfa Aesar) and loaded into a furnace with a 32 mm outer diameter quartz tube. CVD growth was performed at atmospheric pressure using ultrahigh-purity argon as the carrier gas. A second crucible containing 350 mg of sulfur ($\geq$ 99.99% purity, Sigma Aldrich) was located upstream from the growth substrates. Further details of the procedure are described *in Dumcenco et al., 2014, supra.*

*CVD MoS$_2$ transfer from sapphire to SiN$_x$ membrane*

Monolayer MoS$_2$ grown on sapphire substrate (12 mm by 12 mm) is coated by A8 PMMA (495) and baked at 180 °C. A diamond scriber was used to cut it into 4 pieces. Each piece is immersed into 30%w KOH at 85-90°C for the detachment. Capillary force might be used in the interface between polymer and sapphire to facilitate the detachment and reduce the etching time in the KOH. The detached polymer film was repeatedly in DI water. Lastly, the "fishing" method of graphene transfer can be used to transfer CVD MoS$_2$ to the target SiN$_x$ membrane.

*Nanopore forming*

For the nanopore fabrication experiments, after mounting in the polymethylmethacrylate (PMMA) chamber (Figure 1b, step (a)), the chips were wetted with H$_2$O:ethanol (v:v, 1:1) for at least 20 min (Figure 1b, step (b)). 1 M KCl solution buffered with 10 mM Tris-HCl and 1 mM EDTA at pH 8.0 was injected in the chamber (Figure 1b, step (c)). A pair of chlorinated Ag/AgCl electrodes was immersed in the chamber (Figure 1b, step (d)) and employed to apply the transmembrane voltage and the current between the two electrodes was measured by a FEMTO DLPCA-200 amplifier (FEMTO® Messtechnik GmbH). A low voltage (100 mV) was applied to check the current leakage of the membrane. If the leakage current was below 1 nA, the voltage bias was set up in 100 mV steps (25 s for each step) (Figure 1b, step (e)). At a critical voltage (i.e. 800 mV), the current started to immediately increase above the leakage level. A FPGA card and custom-made LabView software was used for applying the voltage and monitoring the conductance (Figure 1b, step (f)). The critical voltage was automatically shut-down by a feedback control implemented in LabView program as soon as the desirable conductance was reached (Figure 1b, step (g)). Nanopores were further imaged using a JEOL 2200FS high-resolution transmission electron microscope (HRTEM). STEM-EDX was performed on a ChemiSTEM-equipped FEI Tecnai Osiris transmission electron microscope (TEM). Aberration corrected TEM micrographs were taken on FEI Titan Themis.

### CVD graphene growth for comparative examples

Large-area graphene films are grown on copper foils. The growth takes place under the flow of a methane / argon / hydrogen reaction gas mixture at a temperature of 1'000°C. At the end of the growth, the temperature is rapidly decreased and the gas flow turned off. The copper foils are then coated by PMMA and the copper etched away, resulting in a cm-scale graphene film ready to be transferred on the chips with membranes for fabricating nanopores with a method of the invention.

### Finite element analysis model

To estimate the potential drop in a defect in a $MoS_2$ membrane a finite element analysis was performed using COMSOL Multiphysics 4.4b. A coupled set of the Poisson-Nernst-Planck equations was solved in a 3D geometry with axial symmetry. In the modeled configuration cis and trans chambers were connected by a 0.3 nm pore in a 0.7 nm thick membrane suspended on a 50 nm wide and 20 nm thick hole. A 0.3 nm diameter defect can correspond to the absence of a unit cell of $MoS_2$. In the model, the applied potential was set to 800 mV and salt concentration was 1 M KCl. The minimal mesh size used was less than 0.2 Å.

### Detailed data analysis of ionic current steps presented in Fig. 5

All analysis were implemented in Matlab R2014b. The raw signal was down-sampled to 5 kHz and then filtered using the edge-preserving Chung-Kennedy (CK) filter *(Chung et al., 1991, J. Neurosci. Methods, 40, 71-86)*. The pore formation in 21 steps, presented on **Fig. 5** can be as follows: the growth of the nanopore is due to sequential cleaving of unit cells from $MoS_2$ lattice. The final pore area is 2.9 $nm^2$, which corresponds to 34 unit cells. Increments in the effective pore size $\Delta A$ are normalized by unit cell size u = 0.0864 $nm^2$. The obtained number $\Delta N = \Delta A/u$ was rounded to the nearest integer, integer +1/3 or integer +2/3 to get $\Delta X$, the number of $MoS_2$ unit cells cleaved during the pore formation process. It is assumed that 1/3 corresponds to a $S_2$ group and 2/3 to a Mo atom, corresponding to the partial cleaving of a unit cell. It should be noted that the two S atoms in $MoS_2$ are stacked vertically and their combined surface area is smaller than that for Mo (which has about 50% larger radius).

The sequence of cleaving $MoS_2$ unit cells and Mo and S atoms in 21 steps to form the pore is given in the table from **Fig. 5e**. In order to depict the sequence of the pore formation, the numbering of the lines in this table and polygons based on HRTEM image starts from 1 to 21. Lifetime of the steps in the sequence is given in the same table. Initially irregular pore gradually becomes more symmetrical. The atom groups have been selected in the manner to minimize the number of dangling bonds at the edge of the pore. The pore formation sequence is not unique, however, the dangling bond constraint significantly reduces the number of pore formation scenarios and induces more symmetrical pore shape.

## Example 2: DNA sensing with nanopores fabricated by a method of the invention

To test the performance of ECR-fabricated pores, DNA translocation experiments were carried and detected the translocation events by the current drops below the baseline current. ECR fabricated $MoS_2$ nanopores consistently produces low-1/*f* noise on the current baseline in the range of 50-100 pA. The major contribution to the 1/f noise in 2D membrane nanopores can be attributed to mechanical fluctuations of the thin membranes. Higher frequency fluctuations are produced by the method itself. Fluctuation noise can be significantly reduced by using a smaller supporting opening, or operating at low temperatures. To show the ability of ECR fabricated nanopore for DNA detection, 2.7 kbp pNEB plasmid DNA (New England Biolabs) is translocated through a relatively large $MoS_2$ nanopore (25 nm) to eliminate the pore-DNA interaction and multiple conformation issues. **Fig. 6a** displays only one-level events indicating an extended (unfolded) DNA conformation, with SNR >10. Scatter plots are used to describe the statistics of DNA translocation as shown in **Fig. 6b.** The signal amplitude also increases linearly with the applied voltage, which is 0.5 nA for 450 mV and 0.38 nA for 300 mV as shown in the histogram **Fig. 6b.** Dwell times are also comparable with DNA translocation through

a TEM-drilled $MoS_2$ nanopore of a similar diameter, for the same DNA and under same bias conditions. In addition, $\lambda$-DNA (48 k bp) is also translocated through an ECR-fabricated nanopore. As expected, folding scenario can be observed, manifested by quantization of current levels guided by dash lines. The upper dash line is the one level conductance, indicating a linear translocation. The bottom dash line is the two level conductance, indicating a folded translocation **(Fig. 7).**

### Nanopore testing for DNA sensing

Current-voltage (I-V) characteristic and DNA translocation were recorded on an Axopatch 200B patch clamp amplifier (Molecular Devices, Inc. Sunnyvale, CA). DNA samples (pNEB193, plasmid 2.7 k bp, New England; $\lambda$-DNA, 48 k bp, New England) were diluted by mixing 10 $\mu$L of $\lambda$-DNA or pNEB stock solution with 490 $\mu$L 1 M KCl buffer. NI PXI-4461 card was used for data digitalization and custom-made LabView software for data acquisition using Axopatch 200B. The sampling rate is 100 kHz and a built-in low-pass filter at 10 kHz is used. Data analysis enabling event detection is performed offline using a custom open source Matlab code, named OpenNanopore (Raillon et al, Nanoscale, 2012, 4, 4916-4924) (http://lben.epfl.ch/page-79460-en.html).

Altogether, those data support that a method of the invention leads to reproducible and fully characterized nanopores by I-V characteristics and size as confirmed by TEM. Further, the intrinsic electrochemical reaction kinetics permits an advantageously high precision for nanopore fabrication at the atom level which can be monitored by the observed step-like features in the ionic current traces. Finally, the nanopores obtained through a method of the invention have demonstrated to allow DNA translocation and their sensing as fully functional nanopores.

### Claims

1. A method for forming a nanopore in a membrane of transition metal dichalcogenide (TMDC) crystals comprising the steps of:

   - providing at least one TMDC thin layer having from about 0.3 nm to 5 nm thickness ($H_m$) suspended in an electrically conducting liquid ;
   - applying a transmembrane voltage (V) at a value slightly higher than the oxidation potential of the transition metal of the said at least one TMDC thin layer configured for an electrochemical atomic etching of said transition metal dichalcogenide thin layer;
   - measuring the ionic current ($I_i$) in the said electrically conducting liquid;
   - turning off the transmembrane voltage once the ionic current ($I_i$) has reached a value ($I_p$) corresponding to the electrical conductance of a pore within the said TMDC thin layer having a prescribed diameter ($d_p$).

2. A method according to claim 1 wherein the TMDC is of chemical formula is $MX_2$, where M is a transition metal atom and X is a chalcogen (S, Se, or Te).

3. A method according to claim 1 the TMDC is selected from $MoS_2$, $SnSe_2$, $WS_2$, $TeS_2$, $MoSe_2$, $WSe_2$, and $TeSe_2$.

4. A method according to any one of claims 1 to 3, wherein TMDC thin layer is a single or double layer.

5. A method according to any one of claims 1 to 4, wherein the TMDC layer comprises $MoS_2$ thin layers or is a $MoS_2$ monolayer.

6. A method according to claim 5 wherein the applied transmembrane voltage is from about 800 mV to about 1'000 mV.

7. A method according to any one of claims 1 to 6, wherein the transmembrane voltage is applied in an essentially constant DC manner.

8. A method according to any one of claims 1 to 7, wherein the ionic current is measured in an ionic current circuit comprising a pair of electrodes, for instance Ag/AgCl electrodes, one of the pair of electrodes located in the electrically conducting liquid on one side of the membrane and the other of the pair of electrodes located in the electrically conducting liquid on the other side of the membrane.

9. A method according to any one of claims 1 to 8, wherein the electrically conducting liquid comprises or consists in an aqueous liquid comprising an electrolyte.

**10.** A method according to any one of claims 1 to 9, wherein the TMDC thin layer is a sensing membrane already integrated in a biosensing device.

**11.** A method according to any one claims 1 to 10 wherein the size of the pore is from about 1 nm to about 5 nm.

**12.** A method according to any one claims 1 to 11, wherein the electrically conducting liquid is an aqueous ionic solution selected from water and KC1 or any inorganic salts such as LiCl, NaCl, $MgCl_2$ $CaCl_2$.

**13.** A method according to any one of claims 1 to 12, wherein the turning off of the transmembrane voltage is achieved by an automatic switch which is activated through a feed-back control circuit when said prescribed electrical conductance current is reached.

**14.** A method according to any one of claims 1 to 13, wherein more than one TMDC thin layers are provided and a transmembrane voltage is applied to each of the TMDC thin layers in parallel.

**15.** A method according for manufacturing a biosensing nanopore device comprising a step of forming a nanopore according to any one of claims 1 to 14.

**a**

**b**

a) MoS$_2$ membrane mounted in a PMMA flow cell at the interface of a cis and trans chamber

↓

b) Wet cis and trans chambers of the flow cell with ethanol/ deionized water (v/v, 1:1) (e.g. for 30 min)

↓

c) Flush both chambers of the flow cell with 1M KCl, pH (5-10)

↓

d) Immerse a pair of freshly made Ag/AgCl electrodes connected to a programmed amplifier in each chamber

↓

e) Apply a step potential from 0 with (20-100mV) increments and hold each step for 25-50 s

↓

f) Measure ionic current while the feedback is set on to zero potential when a preset threshold current for pore size is reached

↓

g) Potential is zeroed and a pore is formed

↓

h) Replace the electrically conducting liquid (e.g. in the *cis* chamber) with another electrically conducting liquid (optional)

Figure 1

Figure 2

Figure 3

Figure 3 continued

Figure 4

Figure 5

e

| I step [nA] | lifetime [s] | D [nm] | A [nm²] | N | ΔA [nm²] | ΔN | ΔX: | atoms/groups cleaved | | | |
|---|---|---|---|---|---|---|---|---|---|---|---|
| 0,926 | 0,1 | 0,36 | 0,10 | 1,16 | 0,100 | 1,16 | 1 | | MoS₂ | | 1 |
| 0,5556 | 0,2 | 0,47 | 0,18 | 2,04 | 0,076 | 0,88 | 1 | | MoS₂ | | 2 |
| 0,4939 | 1,7 | 0,57 | 0,25 | 2,92 | 0,076 | 0,88 | 1 | | MoS₂ | | 3 |
| 0,8025 | 1,0 | 0,71 | 0,39 | 4,53 | 0,139 | 1,60 | 1 2/3 | | MoS₂ | Mo | 4 |
| 0,4939 | 0,1 | 0,79 | 0,49 | 5,62 | 0,094 | 1,09 | 1 | | MoS₂ | | 5 |
| 0,5556 | 2,7 | 0,87 | 0,60 | 6,93 | 0,114 | 1,31 | 1 1/3 | | MoS₂ | S₂ | 6 |
| 0,5556 | 0,8 | 0,96 | 0,72 | 8,34 | 0,122 | 1,41 | 1 1/3 | | MoS₂ | S₂ | 7 |
| 0,8025 | 3,2 | 1,08 | 0,91 | 10,53 | 0,189 | 2,19 | 2 | MoS₂ | MoS₂ | | 8 |
| 0,4322 | 1,5 | 1,14 | 1,02 | 11,79 | 0,109 | 1,26 | 1 1/3 | | MoS₂ | S₂ | 9 |
| 0,3087 | 0,1 | 1,18 | 1,10 | 12,72 | 0,080 | 0,93 | 1 | | MoS₂ | | 10 |
| 0,3087 | 0,1 | 1,23 | 1,18 | 13,67 | 0,083 | 0,96 | 1 | | MoS₂ | | 11 |
| 0,3087 | 1,1 | 1,27 | 1,27 | 14,66 | 0,085 | 0,98 | 1 | | MoS₂ | | 12 |
| 0,6791 | 1,8 | 1,36 | 1,46 | 16,92 | 0,195 | 2,26 | 2 1/3 | MoS₂ | MoS₂ | S₂ | 13 |
| 0,4939 | 0,7 | 1,43 | 1,61 | 18,64 | 0,149 | 1,72 | 1 2/3 | | MoS₂ | Mo | 14 |
| 0,7408 | 2,7 | 1,53 | 1,84 | 21,35 | 0,234 | 2,71 | 2 2/3 | MoS₂ | MoS₂ | Mo | 15 |
| 0,6791 | 0,4 | 1,62 | 2,07 | 23,96 | 0,226 | 2,62 | 2 2/3 | MoS₂ | MoS₂ | Mo | 16 |
| 0,6173 | 1,4 | 1,71 | 2,29 | 26,45 | 0,215 | 2,49 | 2 2/3 | MoS₂ | MoS₂ | Mo | 17 |
| 0,7408 | 0,1 | 1,80 | 2,55 | 29,57 | 0,270 | 3,12 | 3 | MoS₂ | MoS₂ | MoS₂ | 18 |
| 0,3087 | 0,1 | 1,84 | 2,67 | 30,91 | 0,116 | 1,34 | 1 1/3 | | MoS₂ | S₂ | 19 |
| 0,4321 | 0,1 | 1,90 | 2,84 | 32,84 | 0,166 | 1,92 | 2 | MoS₂ | MoS₂ | | 20 |
| 0,2469 | 0,5 | 1,93 | 2,93 | 33,96 | 0,097 | 1,12 | 1 | | MoS₂ | | 21 |
| Pore: | Diameter: | size nm² | cells | | | | | | | | |
| | 1,9 nm | 2,9 | 34,0 | | | 34 | | | | | |

Figure 5 (continued)

Figure 6

Figure 7

Figure 8

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 2014/144818 A2 (HARVARD COLLEGE [US]) 18 September 2014 (2014-09-18) * abstract * ----- | 1-15 | INV. G01N33/487 B81B1/00 |
| A | ITARU YANAGI ET AL: "Fabricating nanopores with diameters of sub-1 nm to 3 nm using multilevel pulse-voltage injection", SCIENTIFIC REPORTS, vol. 4, 21 May 2014 (2014-05-21), XP055200768, DOI: 10.1038/srep05000 * page 2; figure 1 * ----- | 1-15 | |

TECHNICAL FIELDS
SEARCHED    (IPC)

G01N
B81B

The present search report has been drawn up for all claims

| Place of search | Date of completion of the search | Examiner |
|---|---|---|
| The Hague | 9 July 2015 | Steinmetz, Johannes |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 15 15 8894

This annex lists the patent family members relating to the patent documents cited in the above-mentioned European search report.
The members are as contained in the European Patent Office EDP file on
The European Patent Office is in no way liable for these particulars which are merely given for the purpose of information.

09-07-2015

| Patent document cited in search report | Publication date | Patent family member(s) | Publication date |
|---|---|---|---|
| WO 2014144818 A2 | 18-09-2014 | US 2014262820 A1<br>WO 2014144818 A2 | 18-09-2014<br>18-09-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2013167952 A
- WO 2014144818 A
- WO 2012093360 A
- EP 2015053042 W

### Non-patent literature cited in the description

- **BRANTON et al.** *Nature Biotechnology,* 2008, vol. 26, 1146
- **DECKER,.** *Nature Nanotechnology,* 2007, vol. 2, 209
- **PARK et al.** *Small,* 2007, vol. 3, 116-119
- **SIWY et al.** *Physical review letters,* 2002, vol. 89, 198103
- **KWOK et al.** *Plos One,* 2014, vol. 9
- **GARAJ et al.** *Nature,* 2010, vol. 467, 190
- **HULLIGER ; LÉVY.** Structural Chemistry of Layer-Type Phases. Springer, 1976
- **RAILLON et al.** *Nanoscale,* 2012, vol. 4, 4916-4924
- **NOVOSELOV et al.** *PNAS,* 2005, vol. 102, 10541-1053
- **LIU et al.** *Nano Lett.,* 2012, vol. 12, 1538-1544
- **YUAN ; GAOQUAN.** *Nanoporous graphene materials, Materials Today,* 2014, vol. 17 (2), 77-8
- **COHEN-TANUGI D et al.** *Nano Letters,* 2012, vol. 12, 3602-3608
- **BRIGGS et al.** *nanotechnology,* 2015, 084004
- **KOWALCZYK et al.** *Nanotechnology,* 2011, 22
- **FISCHBEIN et al.** *Applied Physics Letters,* 2008, 93
- **LIU et al.** *Nat. Commun.,* 2013, 4
- **BONDE et al.** *Faraday Discussion,* 2008, vol. 140, 219-231
- **LIU et al.** *ACS Nano,* 2014, vol. 8, 2504-2511
- **DUMCENCO et al.** *arXiv preprint arXiv,* 2014, vol. 1405, 0129
- **CHUNG et al.** *J. Neurosci. Methods,* 1991, vol. 40, 71-86
- **RAILLON et al.** *Nanoscale,* 2012, vol. 4, 4916-4924, http://lben.epfl.ch/page-79460-en.html